# EUROPÄISCHE PATENTANMELDUNG

(11) **EP 1 066 851 A1**
(43) Veröffentlichungstag der Anmeldung: **10.01.2001**
(21) Anmeldenummer: 00112879.2
(22) Anmeldetag: 19.06.2000
(51) Int. Cl.: A61M 25/00

(54) **Verfahren zum Einbringen eines Fluides in ein Gefäss des menschlichen Körpers sowie entsprechende Kanüle**

(30) Priorität: 18.06.1999 DE 19928018; 15.07.1999 DE 19933171
(71) Anmelder: MEDOS Medizintechnik AG, 52222 Stolberg (DE)
(72) Erfinder: Reul, H., Dr.-Ing., 52353 Düren (DE); Messmer, Bruno, Prof. Dr. med., 52076 Aachen (DE); Marseille, Oliver, Dipl.-Ing., 52070 Aachen (DE); Kerkhoffs, Wolfgang, Dipl.-Ing., 52080 Aachen (DE); Hildinger, Karl Heinz, 52080 Aachen (DE); Eilers, Rolf, Dr.-Ing., 52222 Stolberg (DE)
(74) Vertreter: Castell, Klaus, Dr.-Ing.

(57) **Zusammenfassung**

Ein Verfahren zum Einbringen eines Fluids in ein Gefäß, zeichnet sich dadurch aus, dass jeder Fluidpartikel bei seinem Eintritt in das Gefäß sowohl eine axiale Bewegung als auch eine Wirbelbewegung ausführt. Hierzu wird eine spezielle Kanüle verwendet, die jedem durch die Kanüle in ein Gefäß eingebrachten Fluidpartikel eine Wirbelbewegung durch einen Drallkörper aufprägt.

## Beschreibung

Die Erfindung betrifft ein Verfahren zum Einbringen eines Fluides in ein Gefäß des menschlichen Körpers sowie eine entsprechende Kanüle. Insbesondere betrifft die Erfindung hierbei das Einbringen eines Fluides, wie beispielsweise Blut, in ein Blutgefäß, vorzugsweise in einen Aortenbogen.

Beim Einbringen von Fluid in Körpergefäße besteht die Gefahr, dass ein aus einer entsprechenden Kanüle austretender Freistrahl eine in der Nähe befindliche Gefäßwand außerordentlich belastet. Insbesondere bei älteren Patienten kann diese Belastung dazu führen, dass sklerotische Ablagerungen von dem Freistrahl abgeschwemmt werden und in den Blutkreislauf gelangen. Hierdurch können unter anderem Embolien bedingt werden.

Um diesem zu begegnen, werden beispielsweise in der US-A-5,354,288 und in der US-A-5,643,226 im Austrittsbereich der Kanüle Prallkörper vorgesehen. Diese können beispielsweise eine Tropfenform oder eine austrittsseitig angebrachte Prallplatte umfassen. Darüber hinaus schlägt die US-A-5,354,288 im Austrittsbereich einen Diffusor vor, der ebenfalls einen zu sehr gerichteten Freistrahl vermeiden soll.

Es ist Aufgabe vorliegender Erfindung, den durch den Freistrahl auftretenden Strahldruck gegenüber dem Stand der Technik weiter zu reduzieren.

Hierzu schlägt die Erfindung einerseits ein Verfahren zum Einbringen eines Fluides in ein Körpergefäß, vorzugsweise in einen Aortenbogen, vor, bei welchem jedes Fluidpartikel bei seinem Eintritt in das Gefäß sowohl eine axiale Bewegung als auch eine Wirbelbewegung ausführt.

Die nach dem Stand der Technik vorgeschlagenen Lösungen können eine derartige Wirbelbewegung nicht allen Fluidpartikeln aufprägen, da die auftretenden Geschwindigkeiten zu hoch sind, als dass bei den verhältnismäßig kurzen Wechselwirkungsstrecken sämtliche Fluidpartikel eine Wirbelbewegung aufgeprägt bekommen können.

Außerdem ist bei den bekannten Einbauten unmittelbar vor dem Eintrittsbereich nachteilhaft, dass es durch die Einbauten zu einer Querschnittsverengung und damit zu einer Erhöhung der Fließgeschwindigkeit kommt. Eine Erhöhung der Fließgeschwindigkeit sollte jedoch grundsätzlich vermieden werden.

Die Erfindung schlägt als weitere Lösung eine Kanüle, insbesondere eine Aortenkanüle vor, die Mittel umfasst, welche jedem durch die Kanüle in ein Gefäß eingebrachten Fluidpartikel eine Wirbelbewegung aufprägen.

Vorteilhaft ist es, wenn der Drallkörper den Querschnitt der Kanüle im Auslassquerschnitt nicht reduziert. Der Drallkörper sollte im Bereich der Kanüle vor einem querschnittreduzierten Durchbruch durch die Gefäßwand angeordnet werden, damit im verengten Eintrittsbereich nicht durch den Drallkörper eine weitere Querschnittsreduzierung erzielt wird.

Insbesondere soll eine derartige Wirbelbewegung bereits bei Eintritt des Fluids in das Gefäß vorhanden sein und nicht erst durch Wechselwirkung mit dem Gefäß bedingt sein.

Vorzugsweise weist der der Wirbelbewegung zuzuordnende Wirbelvektor mit wenigstens einer Komponente in Richtung der übrigen Bewegung, sprich Linearbewegung, des entsprechenden Fluidpartikels. Dieses führt zu einer Neigung des Fluidstrahls sich weit aufzufächern, wodurch der Strahldruck wirksam reduziert wird.

Die entsprechende Wirbelbewegung lässt sich insbesondere durch einen Drallkörper einfach erreichen. Hierbei ist zu gewährleisten, dass der Drallkörper eine ausreichende Wechselwirkungslänge aufweist, so dass sämtliche Fluidpartikel erfasst werden können. Dieses gilt insbesondere, wenn er sich am Ende der Kanüle befindet.

Andererseits kann, in Fließrichtung gesehen, hinter dem Drallkörper eine Ausgleichsstrecke vorgesehen sein. In dieser Ausgleichsstrecke können Fluidpartikel, die bereits eine Wirbelbewegung ausführen, diese auf weitere Fluidpartikel übertragen. Insofern kann auch mit einer kürzeren Wechselwirkungsstrecke bzw. mit einem kürzeren Drallkörper gewährleistet werden, dass sämtliche Fluidpartikel eine Wirbelbewegung aufweisen.

Die Ausgleichsstrecke kann hierbei Merkmale aufweisen, die die Strömungsführung darüber hinausgehend beeinflussen.

Es ist auch möglich, den Drallkörper in Fließrichtung vor einem abgewinkelten Bereich der Kanüle vorzusehen. Dieses gewährleistet einerseits eine bessere Strömungsführung. Andererseits unterscheiden sich verschiedene Kanülen, insbesondere Aortenkanülen, durch ihren abgewinkelten Bereich, während die übrigen Bereiche weitgehend standarisiert und identisch sind. Insofern kann für verschiedene Kanülen ein identischer Drallkörper verwendet werden. Hierdurch lassen sich die Produktionskosten reduzieren.

Es versteht sich, dass der abgewinkelte Bereich als Ausgleichsstrecke genutzt werden kann.

Darüber hinaus kann der Drallkörper in Fließrichtung vor einem sich verjüngenden Bereich der Kanüle vorgesehen sein. Auch dieser bedingt eine bessere Strömungsführung und ein besseres Aufprägen der Wirbelbewegung. Darüber hinaus wird die Rotation durch die Verjüngung beschleunigt, so dass die Wirbelbewegung bei Eintritt des Fluids in das Gefäß wesentlich höher ist.

Es versteht sich, dass ein derartiger, sich verjüngender Bereich vorzugsweise mit der Ausgleichsstrecke und/oder dem abgewinkelten Bereich kombiniert werden kann.

Weitere Vorteile, Ziele und Eigenschaften vorliegender Erfindung werden anhand der Beschreibung anliegender Zeichnung dargestellt, in welcher beispielhaft eine in einen Aortenbogen führende Aortenkanüle dargestellt ist.

Das durch die Kanüle perfundierte Blut wird hierbei durch den Drallkörper in eine Wirbelbewegung versetzt. Im Anschluss hieran verjüngt sich der Kanülenkanal in den abgewinkelten Bereich hinein. Diese Strecke dient darüber hinaus als Ausgleichsstrecke. Bei Eintritt in das Gefäß bzw. den Aortenbogen hat somit jedes Fluidpartikel einerseits eine im wesentlichen lineare Bewegungsrichtung, die durch den Kanülenkanal bedingt ist. Aufgrund der durch den Drallkörper bedingten Wirbelbewegung weist andererseits jedes Fluidpartikel eine Wirbelbewegung auf, die einen Wirbelvektor mit einer zur linearen Bewegung parallelen Komponente umfasst. Diese Wirbelbewegung bedingt eine schnelle Aufweitung des Freistrahls, was zu einer Schonung der Gefäßwand führt. Hierdurch lässt sich insbesondere ein Abschwemmen von sklerotischen Ablagerungen wesentlichen reduzieren.

Zwei Ausführungsbeispiele der Erfindung sind in der Zeichnung dargestellt. Es zeigt
- Figur 1: eine schematische Schnittzeichnung durch eine Kanüle und einen Aortenbogen und
- Figur 2: eine perspektivische Ansicht einer weiteren Kanüle.

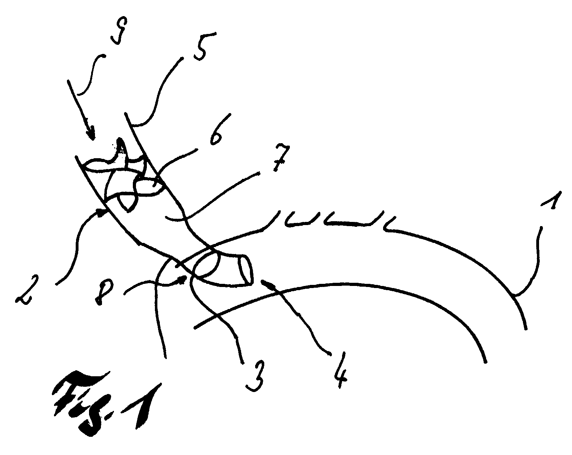

In der Figur 1 ist der Aortenbogen 1 geschnitten dargestellt und in diesen Aortenbogen ist die Aortenkanüle 2 hingeführt. Der ovale Schnittbereich 3 bezeichnet den Durchbruch durch die Gefäßwand des Aortenbogens 1. Der Eintrittsbereich 4 liegt somit innerhalb des Aortenbogens 1 während der hintere Teil 5 der Aortenkanüle 2 außerhalb des Aortenbogens 1 angeordnet ist. Der Eintrittsbereich 4 ist in einem verengten Querschnitt ausgeführt, um den Durchbruch 3 durch die Gefäßwand des Aortenbogens 1 möglichst klein zu halten. Die Verengung des Querschnitts führt darüber hinaus zu einer Erhöhung der Geschwindigkeit im Eintrittsbereich 4. Im nicht verengten Bereich 5 der Kanüle 2 ist ein Drallkörper 6 angeordnet, der durch seine Formgebung dafür sorgt, dass jedem Fluidpartikel zur axialen Bewegung in Richtung der Achse der Aortenkanüle 2 zusätzlich eine Wirbelbewegung, das heißt ein Drall, aufgeprägt wird.

Zwischen dem Drallkörper 6 und dem verengten Eintrittsbereich 4 ist eine Ausgleichsstrecke 7 vorgesehen, die zum Teil einen unreduzierten Querschnitt aufweist und anschließend in den querschnittsreduzierten Eintrittsbereich 4 übergeht.

Etwa im Bereich des Durchbruchs 3 durch die Gefäßwand des Aortenbogens 1 weist die Aortenkanüle einen abgewinkelten Bereich 8 auf, in dem die Aortenkanüle in einem Bogen geführt ist. Die Figur zeigt deutlich, dass der Drallkörper 6 in Fließrichtung 9 vor dem abgewinkelten Bereich 8 angeordnet ist. Da vor dem abgewinkelten Bereich 8 eine Querschnittsverengung vorliegt, ist der Drallkörper in Fließrichtung auch vor dem sich verjüngenden Bereich der Kanüle 2 vorgesehen.

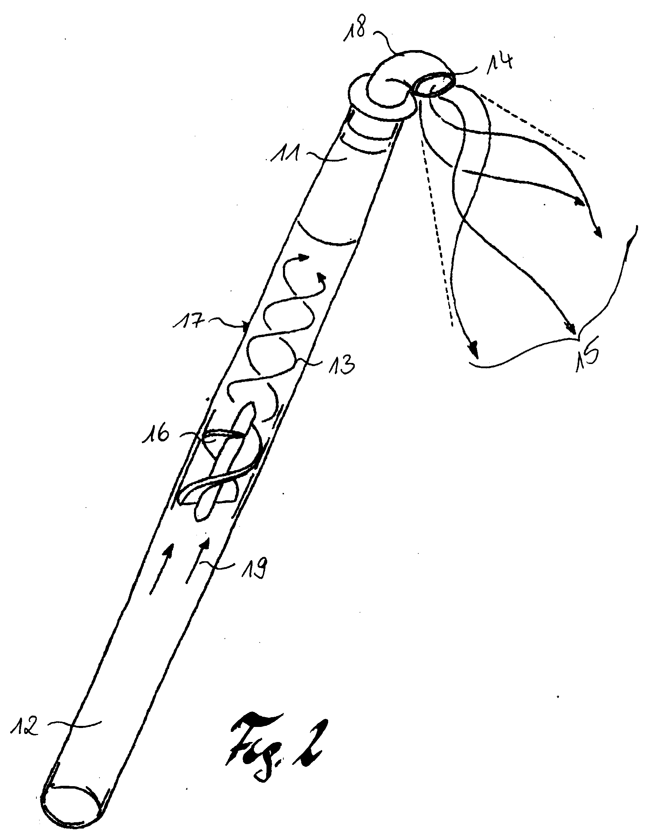

Das Ausführungsbeispiel in Figur 2 zeigt eine Kanüle 12 mit einem Drallkörper 16. Dieser prägt in Fließrichtung 19 dem Fluid eine Wirbelbewegung 13 auf. Danach folgt eine Ausgleichsstrecke 17 und ein sich verjüngender Bereich 11 und anschließend ein abgewinkelter Bereich 18 der Kanüle 12. Die Wirbelbewegung 13 führt zu einer Neigung des Fluidstrahls 15 sich weit aufzufächern, wodurch der Strahldruck beim Verlassen des Eintrittsbereichs 14 wirksam reduziert wird.

## Patentansprüche

1. Verfahren zum Einbringen eines Fluides in ein Gefäß, vorzugsweise in einen Aortenbogen (1), ***dadurch gekennzeichnet, dass*** jedes Fluidpartikel bei seinem Eintritt in das Gefäß (1) sowohl eine axiale Bewegung als auch eine Wirbelbewegung ausführt.

2. Kanüle, insbesondere Aortenkanüle (2), ***gekennzeichnet durch*** Mittel (6), die jedem durch die Kanüle (2) in ein Gefäß (1) eingebrachten Fluidpartikel eine Wirbelbewegung aufprägen.

3. Kanüle nach Anspruch 2, ***gekennzeichnet durch*** einen in der Kanüle (2) befindlichen Drallkörper (6).

4. Kanüle nach Anspruch 3, ***dadurch gekennzeichnet, dass*** der Drallkörper (6) den Querschnitt der Kanüle (2) im Auslassquerschnitt (4) nicht reduziert.

5. Kanüle nach Anspruch 3 oder 4, ***gekennzeichnet durch*** eine in Fließrichtung (9) hinter dem Drallkörper (6) vorgesehene Ausgleichsstrecke (7).

6. Kanüle nach Anspruch 3 bis 5, ***dadurch gekennzeichnet, dass*** der Drallkörper (6) in Fließrichtung (9) vor einem abgewinkelten Bereich (8) vorgesehen ist.

7. Kanüle nach einem der Ansprüche 3 bis 6, ***dadurch gekennzeichnet, dass*** der Drallkörper (6) in Fließrichtung (9) vor einem sich verjüngenden Bereich (3) der Kanüle (2) vorgesehen ist.

8. Verfahren nach Anspruch 1 oder Kanüle nach einem der Ansprüche 2 bis 7, ***dadurch gekennzeichnet, dass*** ein der Wirbelbewegung zuzuordnender Wirbelvektor eine Komponente parallel zur übrigen Bewegung, insbesondere der linearen Bewegung, des Fluidpartikels aufweist.
